(19) Europäisches Patentamt
European Patent Office
Office européen des brevets

(11) Publication number: 0 245 799 B1

# (12) EUROPEAN PATENT SPECIFICATION

(45) Date of publication of patent specification: 23.10.91 Bulletin 91/43

(51) Int. Cl.$^5$: C07F 9/10, G03F 7/00

(21) Application number: 87106719.5

(22) Date of filing: 08.05.87

## (54) Electromagnetic wave-sensitive material and bio-adaptable surface treating agent.

(30) Priority: 13.05.86 JP 107567/86
13.05.86 JP 107568/86
13.05.86 JP 107569/86

(43) Date of publication of application: 19.11.87 Bulletin 87/47

(45) Publication of the grant of the patent: 23.10.91 Bulletin 91/43

(84) Designated Contracting States: BE CH DE FR GB IT LI NL SE

(56) References cited:
EP-A- 0 186 211
ANGEWANDTE CHEMIE, vol. 20, no. 1, 1981, pages 90-91, Verlag Chemie GmbH, Weinheim, DE; A. AKIMOTO et al.: "Polymer model membranes"
ANGEWANDTE CHEMIE, vol. 19, no. 11, 1980, pages 938-940, Verlag Chemie GmbH, Weinheim, US; H.-H. HUB et al.: "Polymerizable phospholipid analogues - New stable biomembrane and cell models"

(56) References cited:
CHEMICAL ABSTRACTS, vol. 107, no. 23, 7th December 1987, page 631, abstract no. 218003c, Columbus, Ohio, US; & JP-A-61 129 190 (NIPPON OILS AND FATS CO., LTD) 17-06-1986
CHEMICAL ABSTRACTS, vol. 106, no. 20, 18th May 1987, page 666, abstract no. 166279e, Columbus, Ohio, US; & JP-A-61 175 081 (CANON K.K.) 06-08-1986

(73) Proprietor: TERUMO KABUSHIKI KAISHA
No. 44-1, Hatagaya 2-chome, Shibuya-ku
Tokyo 151 (JP)

(72) Inventor: Yoshioka, Hiroshi
2517, Ohmiya
Fujinomiya-shi Shizuoka-ken (JP)
Inventor: Suzuki, Kazuhiko
421-3, Kuroda
Fujinomiya-shi Shizuoka-ken (JP)

(74) Representative: Henkel, Feiler, Hänzel & Partner
Möhlstrasse 37
W-8000 München 80 (DE)

EP 0 245 799 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Field of the Invention:

This invention relates to the use of an electromagnetic wave-sensitive material as a bio-adaptable surface treating agent and to the coating of a medical apparatus to rendes it bio-adaptable. More particularly, it relates to the use of an electromagnetic wave-sensitive material having incorporated in a lipid molecule a hydrophobic chain possessing a polymerizable functional group as, a bio-adaptable surface treating agent using a novel polymerizable lipid and permitting formation of a surface suitable for protracted contact with live tissues and body fluids, and for the coating of a medical apparatus possessing a hydrophilic surface of high bio-adaptability.

Description of the Prior Art:

In recent years, electromagnetic wave-sensitive materials such as ultraviolet light-sensitive materials and electron ray-sensitive materials have been attracting attention for their suitability for use in electromagnetic energy substrates, sensors, photoresists, and image recording devices. The electromagnetic wave-sensitive materials heretofore known to the art include polymers having incorporated in the molecules thereof such a sensitive group as an epoxy structure, a carbon-carbon double bond, or an azide group, mixtures of electromagnetic wave-reactive compounds such as bis-azides with polymers possessing a OH group, a COOH group, a $NH_2$ group, or unsaturated bounds and exhibiting virtually or absolutely no reactivity to electromagnetic waves, and such sensitive monomers or oligomers as vinyl monomers or ethylenic oligomers prepared to be used in combination with a polymerization initiator. Oftener than not, however, these electromagnetic wave-sensitive material have low sensitivity to the electromagnetic wave and are impracticable unless they are used in combination with a sensitizer [Japanese Patent Publication SHO 46(1971)-42,363]. In the field of photoresists, for example, the desirability of miniaturization of pattern sizes has been finding growing recognition, developing a need for photoresist materials of high resolving power. In the circumstances, attempts are being made for enhancing resolving power by decreasing the thickness of resist films or for obtaining enhancement of resolving power by superposing an electromagnetic wave fading layer on resist films thereby improving contrast. The electromagnetic wave-sensitive materials embodying outcomes of such attempts, however, are not faultless; those improved by the method which resorts to decreasing the thickness of a resist film are not allowed to lose film thickness amply by reason of the possibility of the film sustaining pinholes on decrease of thickness and those improved by the method which relies on the superposition of a fading layer inevitably require the time of exposure to the electromagnetic wave to be unduly lengthened because the exposure is made through the fading layer. For the electromagnetic wave-sensitive materials of the class described above, it is difficult to acquire satisfactorily improved qualities in terms of sensitivity, contrast and resolving power.

Recently, polymerizable lipids have been proposed as new electromagnetic wave-sensitive materials and various polyacetylene type lipids have been synthesized and are undergoing various studies. Methods for the production of polyacetylene type lipids are disclosed in U.S.P. Nos. 2,816,149, 2,941,014, and 3,065,283, the behavior of these lipids in gelation due to exposure to ultraviolet light is disclosed in H. Ringsdorf et al., Macromol. Chem., 180, 1059 (1979), and the application of such lipids to production of image-forming materials is disclosed in U.S.P. No.4,314,021. Unlike the conventional electromagnetic wave-sensitive materials, the polymerizable lipids acquire a very high resolving power and exhibit high sensitivity and find no use for a polymerization initiator, a sensitizer, or a reducing agent because the lipid monomers are capable of forming a homogeneous and very thin molecular film of fine texture. In the case of the polyacetylene type lipids so far developed, since the conjugated triple bonds in the molecule are synthesized through a number of reaction steps of the nature of pure organic chemistry on the basis of an extremely elaborate molecular design, quantity production of these lipids necessary for the purpose of practical adoption is attained only with great difficulty and, what is more, the electromagnetic wave-sensitive materials finally produced from these lipids turn out to be extremely expensive. Further, the polyacetylene type lipids contain not less than two conjugated triple bonds within the molecule and possess a linear rigid structure, they have the disadvantage that they are not polymerized by virtue of the electromagnetic wave unless they are in the gel phase, i.e. in the crystalline state, at a temperature not exceeding the phase transition. The operation for keeping the reaction system at a sufficiently low temperature costs much.

In the case of such medical devices as artificial internal organs, artificial blood vessels, contact lenses, blood circuits, blood bags, plasma separator, blood tubes, and petri dishes for cultivation of tissues and cells, to ensure safe direct contact of their surfaces with live tissues or body fluids, the medical devices require to possess the so-called bio-adaptability, i.e. the ability to manifest fully their inherent functions as held in contact

with living bodies and, at the same time, refrain from interacting with the live tissues or body fluids. Heretofore, numerous studies have been made on materials and qualities of such medical devices, particularly materials and qualities of the surface portions of such medical devices for the purpose of conferring highly desirable bio-adaptability upon such medical devices, and outcomes of such studies have found utility in practical applications. Examples are negatively charged surfaces formed of anionic polymers or suitably oriented electric polymers, surface coated with natural anticoagulant heparin or synthetic heparin analogs, electrically charged surfaces possessing inherently low surface free energy, and surfaces coated with albumin. These surfaces, however, fail to confer fully satisfactory bio-adaptability upon the medical devices. The reaction of living bodies with such contact surfaces posses a problem yet to be solved. Recently, attention is centering on the idea of utilizing lipids, particularly polymerizable lipids capable of better stability, for conferring bio-adaptability or hydrophilicity upon the surfaces of medical devices in the light of the fact that membranes of living bodies are composed of matrixes of phosphorus-lipid double layers S. L. Regen, Macromolecule, 16, 335 (1983) and Japanese Patent Laid-Open SHO 56(1981)-135,492 . As polymerizable lipids answering the foregoing description, polyacetylene type lipids incorporating conjugated diynes polymerizable functional as groups in their hydrophobic acyl chains have been synthesized and are now being studied extensively. Methods for the production of polyacetylene type lipids are disclosed in detail in the U.S.P. Nos. 2,816,149, 2,941,041, and 3,065,283 and the behavior of such lipids in gelation due to irradiation with ultraviolet light is introduced in detail in H. Ringsdorf et al, Macromolecular Chemistry, 180, 1059 (1979), for example. In the case of the polyacetylene type lipids so far developed, the conjugated diynes in their molecules are synthesized through a number of reaction steps of the nature of pure organic chemistry on the basis of an extremely elaborate molecular design and, that in a poor yield. Thus, they cannot be synthesized in an amount large enough from the commercial point of view and the produced polyacetylene type lipids are prohibitively expensive. Thus, medical devices having their surfaces coated with polymer films of such polyacetylene type lipids are highly restricted both quantitatively and economically. The polymer coats of these polyacetylene type lipids are obtained by polymerizinq the lipids through the agency of a chemical initiator or a varying electromagnetic wave such as ultraviolet light. When the chemical initiator is used, it persists in the final products and possibly gives rise to the problem of toxicity. When the irradiation of the ultraviolet light is adopted, for example, in the production of a catheter, the desired polymer coat cannot be obtained because the ultraviolet light fails to reach the internal surface of the catheter.

This invention, therefore, aims to provide a novel electromagnetic wave-sensitive material. It also aims to provide an electromagnetic wave-sensitive materials which is so sensitive as to be easily polymerized and gelled by irradiation with a varying electromagentic wave such as sunlight, ultraviolet light, β ray, γ ray, and X ray. It further aims to provide an electromagnetic wave-sensitive material using as its principal component a polymerizable lipid which is easy of synthesis, inexpensive, and available abundantly.

This invention has an object of providing a novel bio-adaptable surface treating agent.

Another object of this invention is to provide a bio-adaptable surface treating agent using a novel polymerizable lipid.

Yet another object of this invention is to provide a bio-adaptable surface treating agent capable of polymerizing in the presence of oxygen.

Further, an object of this invention is to rendes a medical apparatus bio-adaptable by using a novel polymerizable lipid.

## SUMMARY OF THE INVENITON

The objects described above are accomplished by this invention in using an electromagnetic wave-sensitive material having as a principal component thereof a polymerizable lipid possessing as a hydrophobic acyl chain thereof at least one acyl chain originating in eleostearic acid and represented by the formula I:

$$CH_3(CH_2)_3CH=CHCH=CHCH=CH(CH_2)_7\overset{\displaystyle O}{\overset{\|}{C}}- \qquad (I)$$

The objects are also accomplished by this invention in making use of an electromagnetic wave-sensitive materials composition formed by dissolving a polymerizable lipid possessing at least one acyl chain originating in eleostearic acid and represented by the formula I in an organic solvent.

Further, the objects are accomplished by this invention rendering a medical apparatus by providing a

hydrophilic surface formed of a polymer coat of a polymerizable lipid possessing as a hydrophobic acyl chain thereof at least one acyl chain originating in eleostearic acid and represented by the formula I.

This invention discloses the use of an electromagnetic wave-sensitive material as a bio-adaptable surface heating agent the using a polymerizable lipid which has the backbone of at least one member selected from phospholipid, sphingolipid, glycolipid, glyceride, glycerol ether, dialkyl phosphate, dialkyl phosphonate, alkyl phosphinate monoalkyl ester, phosphonolipid, N,N-disubstituted dimethyl ammonium halides, trialkyl methyl ammonium halides, tetraalkyl ammonium halides, dialkyl sulfosuccinic esters, and 2,3-diacyloxy succinic acid.

This invention also discloses the use of an electromagnetic wave-sensitive material as a bio-adaptable surface treating agent, and rendering a medical apparatus bio-adaptable using a polymerizable lipid which is an eleostearic acid phospholipid represented by the general formula II:

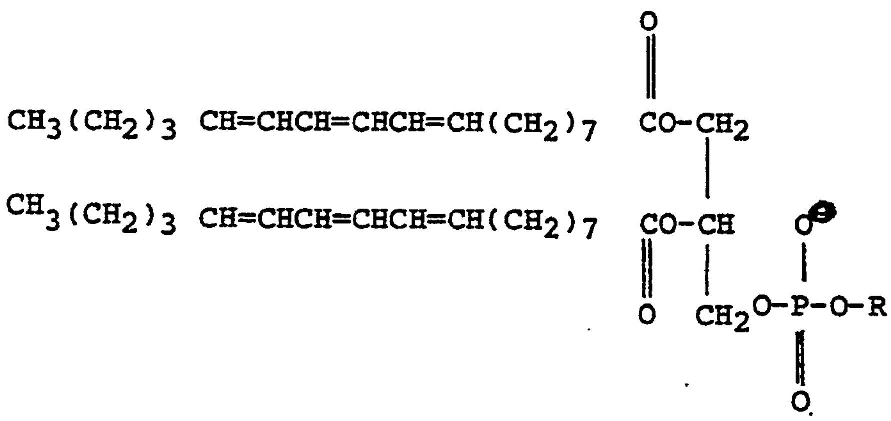

(II)

wherein R stands for $-(CH_2)_2N^{\oplus}(CH_3)_3$, $-(CH_2)_2N^{\oplus}H_3$, or $-CH_2CH(N^{\oplus}H_3)-COO^{\ominus}$. This invention further discloses an electromagnetic wave-sensitive material, a bio-adaptable surface treating agent, and a coated medical apparatus, wherein R in the general formula II is $-(CH_2)_2N^{\oplus}(CH_3)_3$.

This invention provides a medical apparatus having a polymer coat thereof formed by subjecting a polymerizable lipid to cross-linking polymerization through irradiation with an electromagnetic wave and/or by subjecting a polymerizable lipid to cross-linking polymerization through contact with oxygen.

## BRIEF DESCRIPTION OF THE DRAWINGS

Fig. 1 is a chart of an infrared absorption spectrum of one typical material of the present invention, and

Fig. 2 is a chart illustrating a typical ultraviolet light absorption spectrum indicative of the degree of polymerization effected on a liposome formed of the material used according to this invention through irradiation with an ultraviolet light.

## PREFERRED EMBODIMENT OF THE INVENTION

Now, the present invention will be described in detail below with reference to working examples.

The electromagnetic wave-sensitive material used according to the present invention has as a principal component thereof a polymerizable lipid possessing as a hydrophobic acyl chain at least one acyl chain originating in eleostearic acid and represented by the formula I:

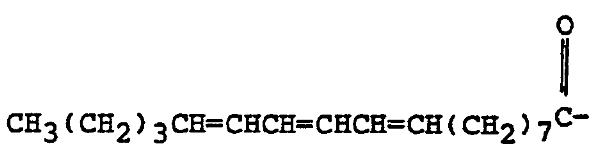

(I)

The medical apparatus rendered bio-adaptable by the present invention possesses at least on the portion thereof destined to contact a living body a hydrophilic surface which is formed of a polymer coat of a polymerizable lipid possessing as a hydrophobic acyl chain thereof at least one acyl chain originating in eleostearic acid and represented by the formula I.

The term "polymerizable lipid" as used in the present specification refers to an amphipatic compound which

has a hydrophilic moiety and a hydrophobic non-polar moiety formed of at least one long-chain aliphatic acyl chain and possessed the backbone of any of such lipids and lipid-related compounds as, for example, phospholipids like phosphatidyl choline, phosphatidyl ethanolamine, phosphatidyl serine, and phoshatidyl glycerol, sphingolipids like sphingomyelin, glycolipids like cerebroside, plant glycolipid, and ganglioside, glycerides like phosphonoglyceride, glycerol ethers, dialkyl phosphates, dialkyl phosphonates, alkyl phosphinate monoalkyl esters, phosphonolipids like ceramid-2-aminoethyl phosphonic acid and phosphonoglyceride, dialkyl phosphates, dialkyl phosphonates, alkyl phosphinate monoalkyl esters, alkyl ammonium halides like N,N'-2-di-substituted dimethyl ammonium halides, trialkyl methyl ammonium halides, and tetraalkyl ammonium halides, dialkyl sulfosuccinicesters, and 2,3-diacyloxysuccinic acids. In the lipids enumerated above, that which has the backbone of an alkyl ammonium halide has a structure such that an acyl chain is bound through the medium of an ester bond to the end or side of the alkyl chain of the compound forming the backbone. The aforementioned designations of lipids of "lipid-related compounds" are meant to indicate the structures destined to constitute the backbones of the lipid monomers in question and therefore, should be interpreted in a sense broad enough to embrace the substituents and similar compounds of such lipids and lipid-related compound. For example, the designation containing a part "alkyl," therefore, embraces all the compounds that answer the designation and severally include such unsaturated hydrocarbon groups as alkenyl, alkadienyl, alkatrienyl, and alkynyl.

The polymerizable lipid possessing at least one hydrophobic acyl chain represented by the formula I and constituting a principal component of the electromagnetic wave-sensitive material and the bio-adaptable surface treating agent of the present invention is one species of the "polymerizable lipds" of the foregoing definition. This polymerizable lipid has the hydrophobic acyl chain thereof synthetically incorporated therein. It is desired, from the standpoint of the impartation of bio-adaptabiliy, to possess among other possible backbones enumerated above the backbone of such a naturally occurring lipid as selected from phospholipids, sphingolipids, glycolipids, glycerol ethers, and phospholipids. Preferably, it is desired to possess the backbone of a phospholipid, a representative biolipid, represented by the formula III:

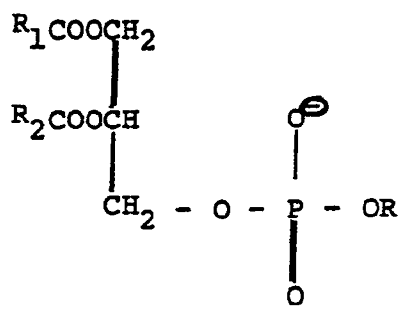

(III)

wherein R stands for $-(CH_2)_2N^{\oplus}(CH_3)_3$ (phosphatidyl choline), $-(CH_2)_2N^{\oplus}H_3$ (cephalin), or $-CH_2CH(N^{\oplus}H_3)-COO^{\ominus}$ (phosphatidyl serine) and $R_1$ and $R_2$ independently stand for a saturated or unsaturated hydrocarbon group, particularly phosphatidyl choline.

The incorporation of the hydrophobic acyl group of the formula I into the lipid possessing the backbone structure can be easily effected by any of the known methods using eleostearic acid as the starting material. This eleostearic acid is a natural unsaturated fatty acid represented by the formula Ia and having conjugated double bonds attached one each to the 9, 11, and 13 positions.

$$CH_3(CH_2)_3CH = CHCH = CHCH = CH(CH_2)_7COOH \quad (Ia)$$

It can be easily extracted from tung oil and accounts for 80 to 95% by weight of mixed fatty acid. The tung oil fatty acid which is obtained by hydrolyzing tung oil contains not less than 60% by weight, preferably not less than 80% by weight, of eleostearic acid and the remaining portion of the acid contains saturated acid, oleic acid, linolic acid, etc. For the production of the electromagnetic wave-sensitive resin and the bio-adaptable surface treating agent of this invention, this tung oil fatty acid can be used in its unmodified form as a natural unsaturated fatty acid. This tung oil fatty acid, when necessary, may be subjected to such a purification treatment as column chromatography and/or recrystallization to permit isolation of eleostearic acid.

The introduction of acyl chains of the formula I from the eleostearic acid into the backbone of a phospholipid, for example, is effected as follows. The hydrophilic polar moiety of lipid which forms the other starting material can be easily and abundantly from any of natural phospholipids (most of which possess a hydrophobic non-polar moiety of the saturated aliphatic acyl chain). The natural phospholipid is hydrolyzed and, in the resultant form of a metal complex such as the complex of such a metal as cadmium, is put to use in the esterification

with the eleostearic acid. This esterification is accomplished by placing the hydrolyzate of the natural phospholipid or the metal complex thereof in a medium such as chloroform, carbon tetrachloride, or methylene dichloride, stirring the medium thereby suspending the hydrolyzate or metal complex therein, adding to the suspension 200 to 400 parts by weight, preferably 300 to 370 parts by weight, based on 100 parts by weight of the hydrolyzate of phospholipid and a suitble amount of a catalyst, displacing the interior of the reaction system with an inert gas such as argon, nitrogen, or helium, and subsequently allowing the reactants to stand for reaction at a temperature in the range of 5° to 40°C, preferalby 15° to 25°C in the range of 5° to 40°C, preferably 15° to 25°C, in a dark place for a period in the range of 24 to 90 hours, preferably 40 to 72 hours. A desirable example of the catalyst used in the reaction is 4-diemthylaminopyridine. The amount of this catalyst used in the reaction is in the range of 50 to 100 parts by weight, preferably 80 to 85 parts by weight, per 100 parts by weight of the hydrolyzate of phospholipid. This reaction terminates with the precipitation of a white insoluble substance. This reaction mixture is filtered to remove the insoluble substance, distilled under a vacuum at room temperature to expel the solvent, dissolved again in a mixed solvent containing chloroform, methanol, and water (at a volumetric ratio of 4 : 5 : 1), brought into contact with an ion-exchange resin, and eluted from the ion-exchange resin. The eluate is distilled under a vacuum to expel the mixed solvent, dissolved in a small amount of chloroform and then purified as in a silica gel column with a mixed solvent of chloroform and methanol, to obtain eleostearic acid phospholipid represented by the general formula II:

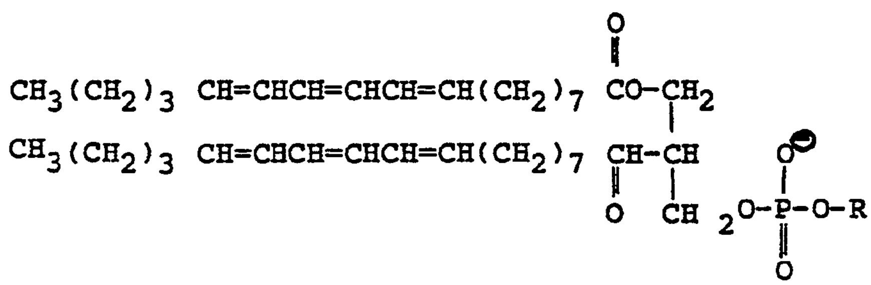

(II)

wherein R stands for $-(CH_2)_2N^{\oplus}(CH_3)_3$, $-(CH_2)_2N^{\oplus}H_3$, or $-CH_2CH(N^{\oplus}H_3)-COO^{\ominus}$.

The material thus obtained varies with the kind of starting material used. When lecithin of the yolk is used as the starting material, for example, there is obtained eleostearic acid phosphatidyl choline represented by the formula IV. When cephalin or phosphatidyl serine is used as the raw material, there is obtained a corresponding electromagnetic wave-sensitive material.

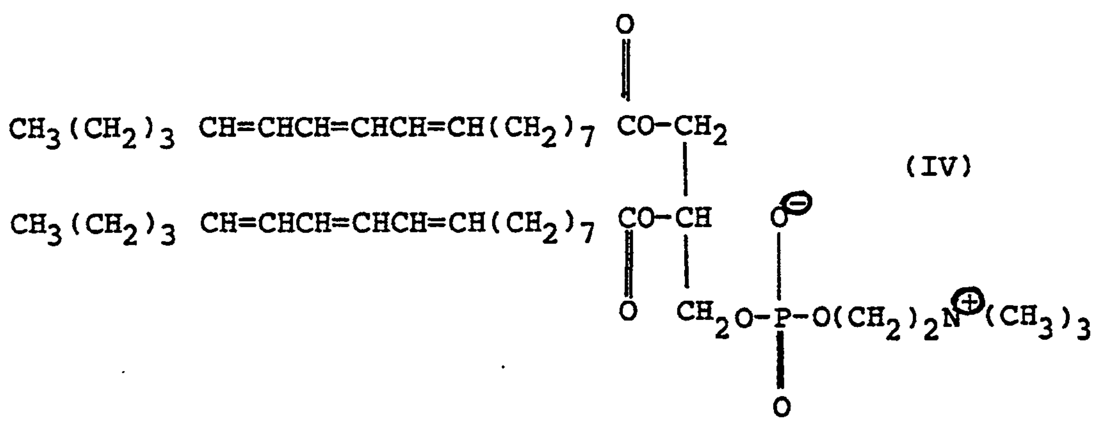

(IV)

Besides the polymerizable lipid of the foregoing description, the material of this invention can contain such additives as a coating aid, a stabilizer, a buffer, and a chelating agent, a hydrophilic binder such as gelatin, polyvinylalcohol, poly-(N-vinyl-2-pyrrolidone), polyacrylamide, an acrylamide type copolymer, an acryl type polymer, or an acryl type copolymer, or a diluent.

The electromagnetic wave-sensitive material used as bio-adaptable treating agent according to the present invention uses as its principal component the polymerizable lipid having an acyl chain originating in an eleostearic acid and possessing three conjugated double bonds in the chain as represented by the formula I. When this material is exposed to a varying electromagnetic wave such as sunlight, ultraviolet light, β ray, γ ray, or X ray, particularly to the ultraviolet light, the three conjugated double bonds in the hydrophobic acyl chain are readily caused to undergo cross-linking polymerization, with the result that the polymerizable lipid is

polymerized and gelled to assume a stable state. This conjugated triene type polymerizable lipid has the wavelength of maximum in absorption spectrum at a relatively low energy position above 270 nm (Fig. 2). Since the polymerizable lipid polymerizes itself with the energy of light, it has no use for a polymerization initiator, a sensitizer, or a reducing agent and, therefore, has no possibility of entailing the problem of toxicity otherwise induced by use of the additives. As compared with the conventional polyacetylene type lipid which is allowed to polymerize only in a crystalline state obtainable at temperatures below the gel-liquid phase transition temperature, the conjugated triene type polymerizable lipid of this invention is caused to undergo polymerization by means of electromagnetic wave in any state either above or below the phase transition temperature because the hydrophobic acyl chain is relatively flexible.

The electromagnetic wave-sensitive material of this invention is soluble in chloroform, ether, methanol or dimethyl formamide before it is irradiated with the electromagnetic wave. The portion of this electromagnetic wave-sensitive material which is polymerized and gelled by irradiation with the electromagnetic wave is perfectly insoluble in such solvents as mentioned above. Thus, the irradiation brings about a remarkable difference in solubility. The polymerizable lipid is used as dissolved in a varying organic solvent such as chloroform, ether, methanol, ethanol, isopropanol, or other similar lower alkanol, or methyl formamide. The concentration of the polymerizable lipid in the resultant solution is in the range of 0.1 to 20% by weight, preferably 0.5 to 10% by weight.

The electromagnetic wave-sensitive material as used according to this invention can form a monomolecular film when it is left spreading on the surface of water. When the material in this state is irradiated with the ultraviolet light, the polymerization proceeds in the material in the form of the monomolecular film. As the result, there can be obtained an extremely thin macromolecular film. An aggregate having such monomolecular films superposed orderly can be obtained by the Langmuir-Blodgett (LB) Method. Since this extremely thin film has an unusually high resolving power, it can be expected to find utility in application to insulating films of IC and LSI, to field of electronic, and even to molecular devices.

Surprisingly it has been found that the conjugated triene type polymerizable lipid, only when it is left standing in the air, is allowed to undergo polymerization. This means that this polymerizing lipid, in the presence of oxygen, automatically sets polymerizing and, as the result,forms a stable cross-linked polymer in much the same way as it is irradiated with the electromagnetic wave. The polymerization of the lipid on the surface such as the inner surface of a catheter which is not easily reached by the ultraviolet light, therefore, can be success fully attained by allowing the surface to stand in the presence of oxygen.

The polymerizable lipid which is used as the bio-adaptable surface treating agent of the present invention is soluble in chloroform, ether, methanol or dimethyl formamide before it is irradiated with the electromagnetic wave and/or exposed to oxygen. Once it is polymerized and gelled by the exposure to the electromagnetic wave and/or oxygen, it is completely insoluble in the solvents mentioned above. The cross-linking polymerization caused in the polymerizable lipid brings about a remarkable difference in solubility. Similarly to the electromagnetic wave-sensitive material, this bioadaptable surface treating agent is put to use as dissolved in an organic solvent. The solvents usable for this solution and the concentration of the polymerizable lipid in the resultant solution are the same as those mentioned above.

Fortunately, since the material as used according to this invention is formed of the polymerizable lipid possessing amphipaticity, the hydrophobic non-polar moiety, namely the acyl chain portion, of the polymerizable lipid is oriented and enabled to exhibit fine adhesiveness on the surface of the hydrophobic substrate and the hydrophilic polar moiety of the lipid is oriented outwardly. Thus, the surface treated with the agent is vested with hydrophilicity. The conjugated triene group which is a sensitive group for inducing the cross-linking polymerization is present in the acyl chain. After the cross-linking polymerization has occurred, therefore, the nature of the hydrophilic polar moiety of the lipid remains intact.

When the polymerizable lipid is dispersed in an aqueous solution as by means of ultrasonic wave, it automatically forms liposomes, i.e. a mass of minute droplets of a fatty double layer structure. In the liposomes, the hydrophobic groups are oriented inside and the hydrophilic groups outside. Even when the polymerizable lipid is in the form of liposomes, the polymerization is induced by the exposure to the electromagnetic wave and/or oxygen. In this case, the advance of this polymerization can be traced by keeping observation of the ultraviolet absorption spectrum of the suspension of liposomes and keeping record of the decrease of absorbance in the ultraviolet light absorption spectrum due to the conjugated triene.

The bio-adaptable surface treating agent as used according to the present invention serves for improving the surface qualities of various medical devices such as artificial internal organs, artificial blood vessels, contact lenses, blood circuits, plasma separators, blood tubes, and petri dishes for the cultivation of tissues or cells. The deposition of a coat of this bio-adaptable surface treating agent on the surface of a blank piece of a given medical device can be effected by any of various known methods. To be short, this deposition may be accomplished by applying the solution or the liposome suspension of this polymerizable lipid on a given

substrate and then expelling the solvent or dispersant from the applied coat of the solution or suspension by evporation. Alternately, it may be attained by superposing on the surface of the substrate a number of monomolecular films of the polymerization lipid formed on the surface of water by the Langmuir-Blodgett (LB) method. By subjecting the polymerizable lipid thus deposited on the substrate to cross-linking polymerization by exposure to the electromagentic wave and/or oxygen, the bio-adaptable surface treating agent can be fixed on the substrate.

The medical apparatus as provided bio-adaptable by this invention is any of the medical devices having the possibility of contacting living bodies, i.e. tissues or body fluids. Examples of such medical devices include artificial internal organs, artificial blood vessels, contact lenses, blood circuits, blood bags, plasma separators, blood tubes, catheters, and petri dishes for the cultivation of tissues or cells. Of course, these are not the only medical devices to which this invention can be applied. The materials forming such medical devices are not specifically defined. They may be natural and synthetic rubber, natural and synthetic fibers, glasses, metals, and ceramics. This invention does not discriminate the surfaces of these medical devices by their hydrophobicity or hydrophilicity.

When a given medical device has a hydrophobic surface, since the polymerizable lipid is an amphipatic compound, the hydrophobic non-polar moiety, namely the acyl chain portion, of the polymerizable lipid is oriented on the surface and allowed to exhibit a fine adhesive property and the hydrophilic polar moiety thereof is oriented externally. Thus, the treated surface can be vested with hydrophilicity. Since the conjugated triene group which is a sensitive group for inducing the cross-linking polymerization is present in the acyl chain, the nature of the hydrophilic polar moiety of the polymerizable lipid remains intact even after the polymer coat is formed in consequence of the cross-linking polymerization. The polymer coat to be formed on the surface of a given medical device can be obtained in the form of a monomolecular film of the polymerizable lipid or in the form of an aggregate of a number of such monomolecular films superposed as by the Langmuir-Blodgett (LB) method. Alternately, the polymer coat may be obtained in the form of liposomes. When the polymerizable lipid of the foregoing description is dispersed in an aqueous solvent by means of ultrasonic wave, for example, it automatically forms liposomes, i.e. small fatty droplets of double layer structure. In these liposomes, the hydrophobic groups of the polymerizable lipid are oriented inside and the hydrophilic grous thereof outside. The outer surface of the liposomes, therefore, assumes hydrophilicity. Since gravitational force of a sort acts on two liposomes, aggregation of such liposomes can form a film. The polymerizable lipid, even in the form of such liposomes, can be allowed to induce polymerization by exposure to the electromagnetic wave and/or oxygen.

Various methods are conceivable as means for providing the the medical apparatus bio-adaptable this invention. To be simple, it can be produced by applying the solution or liposomes suspension of the polymerizable lipid on the surface of at least the portion of a blank piece of a given medical device and expelling the solvent or dispersant from the applied coat of the solution or suspension by evaporation. Alternately, the production may be attained by causing a number of monomolecular films of the polymerizable lipid formed on the surface of water to be superposed on the surface of the blank piece by the LB method. When the coat of the polymerizable lipid deposited on the surface is subjcted to cross-linking polymerization by exposure to the electromagnetic wave and/or oxygen, the polymer coat of the lipid can be fixed on the surface. Particularly the polymer coat obtainable by the cross-linking polymerization through exposure to oxygen can be successfully formed even when the given medical device is something like a catheter and, therefore, the inner surface thereof destined to contact a living body cannot be easily irradiated with the electromagnetic wave such as ultraviolet light and the polymer coat to be formed by the action of the electromagnetic wave cannot be formed on the inner surface or when the given medical device is formed of a material susceptible of deterioration by the electromagnetic wave.

The medical apparatus rendered bio-adaptable by the present invention which is obtained as described above possesses at least in the portion thereof intended for contact with a living body a hydrophilic surface formed of the polymer coat of a polymerizable lipid having as a hydrophobic acyl chain at least one acyl chain originating in eleostearic acid and represented by the formula I. Though the structure of the polymer coat remains yet to be elucidated, this polymer coat is formed of a polymerizable lipid possessing the same structure as the phospholipid which is one of the components of the living membrane. The polymer coat enjoys high bio-adaptability because of its hydrophilic surface and retains a stable state because of its cross-linked structure. Thus, the polymer coat is not degenerated by contact with a living body or with a solvent, for example.

Now, the present invention will be described more specifically below with reference to working examples of the invention.

Example:

Production of elostearic acid anhydride

The amount of tung oil fatty acid equivalent to 80 g of eleostearic acid was dissolved in 600 ml of carbon tetrachloride fresh from the treatment of dehydration and distillation. The resultant solution and 32.6 g of dicyclohexyl carbodiimide added thereto, with the container hermetically sealed after displacement of the inner gas thereof with nitrogen gas, were left standing with occasional agitation at 25°C for 24 hours. The resultant reaction mixture was filtered to remove the insolubles and the filtrate was distilled to dryness. When the dry distillate as purified with silica gel using dichloromethane solvent, eleostearic anhydride was obtained in a yield of 29%.

Production of cadmium complex of yolk lecithin (phosphatidyl choline) hydrolyzate

In 450 ml of dehydrated ether, 45 g of yolk lecithin (Kewpie PL-100) was dissolved. The resultant solution was filtered to remove insolubles. The filtrate and 50 ml of a 10% methanol solution of tetrabutyl ammonium hydroxide werevigorously shaken at a temperature of 25°C. The reaction solution grew cloudy with white suspension and gradually induced layer separation with the advance of the reaction. Then it was left standing to permit thorough precipitation of a brown oily substance. The supernatant was separated by decantation. The brown oily precipitate was washed three times with 100 ml of dehydrated ether. Then the brown only residue was dissolved in 125 ml of dehydrated methanol, and refluxed at the boiling point and hot filtered in the presence of 1 g of a decolorizing agent. The filtrate was cooled and mixed with 250 ml of dehydrated ether to induce precipitation. The supernatant was removed by decantation and the precipitate was dissolved in 40 ml of hot water. The solution of the precipitate and a solution of 8 g of a 5/2 hydrate of cadmium chloride in 20 ml of pure water were refluxed at the boiling point in the presence of 2.5 g of activated carbon and 2 g of a decolorizing agent. The resultant solution was passed through a filter paper and a 0.25 µ m Millipore filter. The filtrate, on addition of 100 to 150 ml of ethanol, gave rise to a colored precipitate. The clouded solution was separated by removing this colored precipitate. When the solution and 100 to 150 ml of ethanol were vigorously shaken, there ensued precipitation of white crystals. The mixture was left standing at a temperature of 0° to 5°C overnight and then filtered to gather the precipitated crystals. The crystals were washed with dehydrated methanol, dehydrated ether, and dehydrated benzene in the order mentioned. The washed crystals were vacuum dried overnight over phosphorus pentoxide at a temperatue of 80°C. Consequently, a cadmium complex of phosphatidyl choline hydrolyzate was obtained in a yield of 56%.

Production of polymerizable lipid by esterification

In 160 ml of chloroform fresh from distillation, 6.74 g of cadmium complex of the yolk lecithin hydrolyzate was agitated to be suspended therein. The resultant suspension, 24.70 g of a tung oil fatty acid anhydride, and 5.61 g of 4-dimethylaminopyridine as a catalyst, with the container tightly stoppered after displacement of the inner gas thereof with argon gas, were agitated for reaction in a dark place at a temperature of 25°C for 60 hours. During this agitation, there occurred precipitation of white insolubles. The reaction mixture was filtered to remove the white precipitate and then distilled under a vacuum at room temperatuer to expel the solvent. The residue of the distillation was dissolved in 100 ml of a mixed solution consisting of methanol, chloroform, and water at a ratio of 5 : 4 : 1. The resultant solution was filtered again and the filtrate was poured into a column of an ion-exchange resin, AG-501-X8(D) (Bio-Rad). The adsorbate was eluted with 500 ml of the aforementioned mixed solvent. The eluate was vacuum distilled at a temperature of 25°C, dissolved again in chloroform, and then purified over a silica gel column. Consequently, eleostearic acid phosphatidyl choline was obtained in a yield of 30%. The infrared absorption spectrum was as shown in Fig. 1.

Production of liposomes from polymerizable lipid

In 6 ml of chloroform, 200 mg of eleostearic acid phosphatidyl choline was dissolved. The lipid solution consequently obtained was placed in a flask of the shape of an eggplant and treated with an evaporator for thorough removal of the solvent and consequent formation of a lipid film on the bottom surface of the flask. The lipid film and 10 ml of a Hepes buffer (10 mM, pH 8.0) were shaken with a vortex mixer and then treated with a chip type ultrasonic projector (40 to 50 W) under a current of argon gas for 10 minutes. The liquid produced by this treatment turned from a whitish turbid state to a clear dispersion, indicating the formation of liposomes. Under a scanning electron microscope these liposomes were found to comprise spherical particles

0.2 to 0.5 μ m in diameter.

Example of polymerization of liposomes, bio-adaptable surface treating agent, by irradiation with electromagnetic wave

In a water bath kept at a temperature of 25°C, a sample held therein in a concentration of 10 mg/ml was irradiated with the ultraviolet light from as 75-W mercury vapor lamp kept at an irradiation distance of 12 cm under a deaerated condition. With the elapse of the irradiation time, the absorbance at 272 nm due to the triene decreased as shown in Fig. 2, indicating the progress of polymerization.

Example of polymerization of bio-adaptable surface treating agent with oxygen

In a flask of an eggplant shape, 100 ml of a chloroform solution containing 500 mg of the eleostearic acid phosphatidyl choline obtained in the preceding example was vacuum distilled with a rotary evaporator to expel chloroform and to obtain a thin film of eleostearic acid phosphatidyl choline on the inner surface of the flask. The film was left standing in a dark place under an atmosphere of air at room temperature for one week. Owing to the oxygen in the air, the eleostearic acid phosphatidyl choline was polymerized and gelled and consequently became perfectly insoluble in such organic solvents as chloroform, ether, and methanol, and in water.

Impartation of hydrophilicity to hydrophobic substrate

A 1 wt% methanol solution of the eleostearic acid phosphatidyl choline obtained in the preceding method was applied on a petri dish made of polystyrene and intended for cultivation of tissues and then dried. Under an atmosphere of air, this petri dish was irradiated with the ultraviolet light from a 75-W mercury vapor lamp at room temperature for six hours. The petri dish was thoroughly washed with distilled water. Then, the angle of contact of a water drop with the surface of the petri dish was measured. This angle of contact on the surface of the petri dish treated as described above was 21°, while the angle of contact on a control petri dish which had not undergone the treatment for the formation of a polymer coat of eleostearic acid phosphatidyl choline was 66°.

Confirmation of bio-adaptability

A 1 wt% methanol solution of the eleostearic acid phosphatidyl choline obtained in the foregoing example was applied on a petri dish of polystyrene for cultivation of tissues and then dried. Under an atmosphere of nitrogen, this petri dish was irradiated with the ultraviolet light from a 75-W mercury vapor lamp at room temperature for 12 hours. On this petri dish, 5 ml of a Hela-S3 cell suspension prepared in advance at a concentration of 1 x $10^5$ cells/ml on a MEM medium was inoculated and cultured for 48 hours. After the elapse of the 48 hours' cultivation, the surface of the pertri dish was observed under a microscope. Consequently, the cells were found to have been established, extended, and propagated.

Confirmation of insoluble coat

A Hepes buffer containing 1 wt% of the eleostearic acid phosphatidyl choline obtained in the aforementioned example in a suspended state was applied on a petri dish of glass and then dried. The applied coat of the suspension on the petri dish was irradiated with the ultraviolet light from a 75-W mercury vapor lamp under an atmosphere of air at room temperature for six hours. It was confirmed that ,as the result of the irradiation, a coat perfectly insoluble in such organic solvents as chloroform, ether, and methanol and in water was formed on the surface of the petri dish.

Separately, in a water bath kept at a temperature of 25°C, the liposomes held therein in a concentration of 10 mg/ml was irradiated with the ultraviolet light from a 75-W mercury vapor lamp kept at an irradiation distance of 12 cm under a deaerated condition. With the elapse of the irradiation time, the absorbance at 272 nm due to the triene was observed to decrease as shown in Fig. 2, indicating the progress of polymerization.

As described above, this invention is directed to the use of an electromagnetic wave-sensitive material which has, as its principal component,as polymerizable lipid possessing as a hydrophobic acyl chain at least one acyl chain originating in eleostearic acid represented by the formula I. The electromagnetic wave-sensitive material, therefore, has high sensitivity and is easily polymerized and gelled on exposure to a varying electromagnetic wave such as sunlight, ultraviolet light, β ray, γ ray, and X ray. It is highly suitable as materials for electromagnetic wave energy substrates, sensors, photoresists, and picture recording elements. It can be

easily synthesized from a starting material which occurs abundantly in nature and can be supplied at a low price in a large quantity. Further, since the sensitive site of the polymerizable lipid forming the electromagnetic wave-sensitive material of this invention possesses a conjugated triene structure, the polymerizable lipid has a flexible molecular structure as compared with the conventional polyacetylene type lipid. This polymerizable lipid, therefore, is allowed to start polymerization by exposure to the electromagnetic wave without reference to the state to be assumed above or below the phase transition temperature. Unlike the reaction system using the polyacetylene type lipid, the reaction system using the polymerizable lipid of this invention is not required to be kept at a low temperature below the phase transition temperature. Moreover, the polymerizable lipid can be spread out on the surface of water, for example, to form an extremely thin monomolecular film thereon and, consequently, to acquire an unusually high resolving power.

The effects described above are manifested more advantageously when the polymerizable lipid possesses the backbone of any member selected from phospholipid, sphingolipid, glycolipid, glyceride, glycerol ether, dialkyl, phosphate, dialkyl phosphonate, alkyl phosphinate alkyl ester, phosphonolipid, N.N-disubstituted dimethyl ammonium halides, trialkyl methyl ammonium halides, tetraalkyl ammonium halides, dialkyl sulfosuccinic esters, and 2,3-diacyloxy succinic acid and when the polymerizable lipid is an eleostearic acid phospholipid represented by the general formula II, particularly eleostearic acid phosphatidyl choline.

The bio-adaptable surface treating agent and the coating of the medical apparatus have substantially the same structure as the component forming a living membrane and exhibit high bio-adaptability and very rarely interact with tissues or body fluid held in contact therewith. Since the bio-adaptable surface treating agent can be synthesized easily, abundantly, and inexpensively from naturally occurring eleostearic acid and naturally occurring lipids as starting materials and can be advantageously utilized as surface treating agent for such medical devices as artificial internal organs, artificial blood vessels, contact lenses, blood circuits, blood bags, plasma separators, blood tubes, and petri dishes for cultivation of tissues or cells. The polymerizable lipid which forms the bio-adaptable surface treating agent can easily be set polymerizing not only by exposure to a varying electromagnetic wave such as sunlight, ultraviolet light, $\beta$ ray, $\gamma$ ray, and X ray but also in the presence of oxygen. The aforementioned bio-adaptable surface treating agent, therefore, is polymerized and gelled to produce a stable film only when it is left standing in the air for a fixed length of time. Unlike the conventional polyacetylene type lipid, this bio-adaptable surface treating agent can be effectively utilized in the portion such as the inner surface of a catheter which is not reached by the electromagnetic wave such as ultraviolet light and in the portion using a material susceptible to deterioration by the action of the electromagnetic wave. Better still, when the bio-adaptable surface treating agent is applied to a substrate having a hydrophobic surface, the treated surface can be vested with hydrophilicity because the hydrophobic acyl chain side of the polymerizable lipid is oriented in the directiion of the surface of the substrate and the hydrophilic polar moiety of the lipid is oriented outwardly.

All these effects are manifested more advantageously when the polymerizable lipid possessing at least one acyl chain originating in eleostearic acid and represented by the formula I is an eleostearic acid phospholipid, particularly eleostearic acid phosphatidyl choline.

## Claims

1. Use of an electromagnetic wave-sensitive material as a bio-adaptable surface-treating agent composition comprising a polymerizable lipid possessing as a hydrophobic acyl chain thereof at least one acyl chain derived from eleostearic acid and represented by the formula I:

$$CH_3(CH_2)_3CH{=}CHCH{=}CHCH{=}CH(CH_2)_7\overset{O}{\overset{\|}{C}}- \qquad (I)$$

2. Use of a material according to Claim 1, wherein said polymerivable lipid has the backbone of at least one member selected from phospholipid, sphingolipid, glycolipid, glyceride, glycerol ether, dialkyl phosphate, dialkyl phosphonate, alkyl phosphinate alkyl ether, phosphonolipid, N,N-disubstituted dimethyl ammonium halides, trialkyl methyl ammonium halides, tetraalkyl ammonium halides, dialkyl sulfosuccinic esters and 2,3-diacyloxy succinic acid.

3. Use of a material according to claim 1, wherein said polymerizable lipid is an eleostearic acid phospholopid represented by the general formula II:

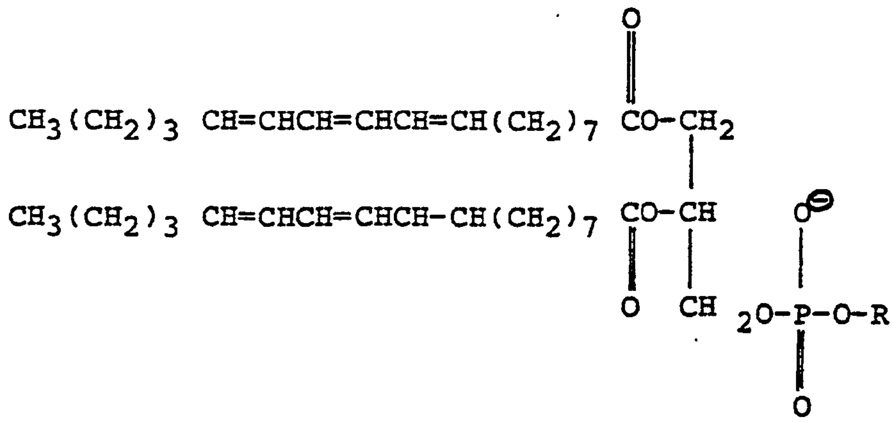

(II)

wherein R stands for $-(CH_2)_2-N^{\oplus}(CH_3)_3$, $-(CH_2)_2-N^{\oplus}H_3$ or $-CH_2CH(N^{\oplus}H_3)-COO^{\ominus}$.

4. Use of a material according to Claim 3, wherein R in the general formula II is $-(CH_2)_2-N^{\oplus}(CH_3)_3$.

5. Use of a material according to one of the preceding claims in the form of a composition obtained by dissolving the said material in an organic solvent.

6. Use of a material according to Claim 5, the concentration of said polymerizable lipid in the organic solvent falling in the range of 0.1 to 20%.

7. Use of a material according to Claim 5, the organic solvent being a lower alkanol.

8. Use of an electromagnetic wave-sensitive material in the form of an electromagnetic wave-sensitive sheet having the surface of a substrate thereof coated with a composition according to one of claims 5 to 7.

9. Use of a material according to Claim 8, wherein the polymerizable lipid is as defined in claim 2.

10. Use of a material according to Claim 8, wherein the polymerizable lipid is as defined in claim 3.

11. Use of the material according to Claim 8, wherein R in the general formula II of claim 3 is $-(CH_2)_2-N^{\oplus}(CH_3)_3$.

12. Use of an electromagnetic wave-sensitive material according to one of Claims 1 to 4 for the coating of a medical apparatus to render it bio-adaptable.

13. Use of a material according to Claim 12, wherein a polymer coat is formed by subjecting said material to cross-linking polymerization through irradiation with an electromagnetic wave and/or through contact with oxygen.

## Patentansprüche

1. Verwendung eines für elektromagnetische Wellen empfindlichen Materials als biokompatibles Oberflächenbehandlungsmittel, umfassend ein polymerisierbares Lipid, das als eine hydrophobe Acylkette mindestens eine von Eleostearinsäure herrührende und durch die Formel I:

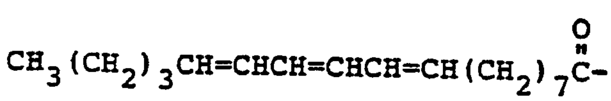

(I)

darstellbare Acylkette enthält.

2. Verwendung eines Materials nach Anspruch 1, worin das polymerisierbare Lipid das Rückgrat mindestens eines aus Phospholipid, Sphingolipid, Glycolipid, Glycerid, Glycerinether, Dialkylphosphat, Dialkylphosphonat, Alkylphosphinatalkylether, Phosphonolipid, N,N-disubstituierten Dimethylammoniumhalogeniden, Trialkylmethylammoniumhalogeniden, Tetraalkylammoniumhalogeniden, Dialkylsulfobernsteinsäureestern und 2,3-Diacyloxybernsteinsäure ausgewählten Bestandteils aufweist.

3. Verwendung eines Materials nach Anspruch 1, worin das polymerisierbare Lipid aus einem Eleostearinsäurephopholipid der allgemeinen Formel II:

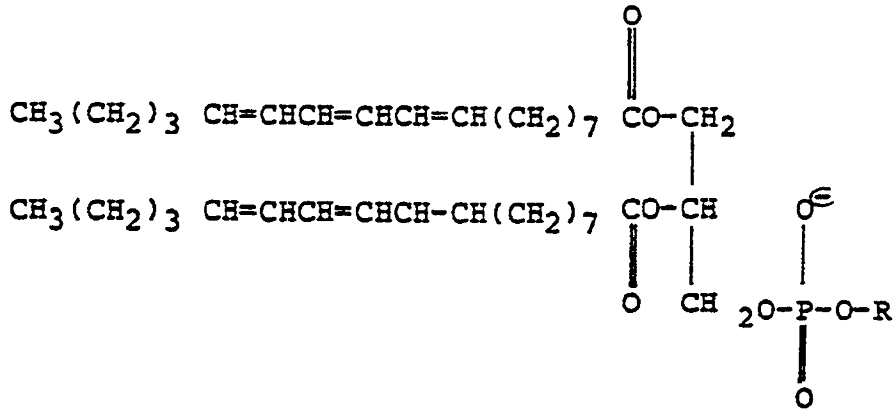

(II)

worin R für $-(CH_2)_2N^{\oplus}(CH_3)_3$, $-(CH_2)_2^{\oplus}N_3$ oder $-CH_2CH(N^{\oplus}H_3)-COO^{\ominus}$ steht, besteht.

4. Verwendung eines Materials nach Anspruch 3, worin R der allgemeinen Formel II $-(CH_2)_2N^{\oplus}(CH_3)_3$ ist.

5. Verwendung eines Materials nach einem der vorhergehenden Ansprüche in Form einer durch Auflösen des Materials in einem organischen Lösungsmittel erhaltenen Masse.

6. Verwendung eines Materials nach Anspruch 5, wobei die Konzentration des polymerisierbaren Lipids in dem organischen Lösungsmittel in den Bereich von 0,1 - 20% fällt.

7. Verwendung eines Materials nach Anspruch 5, worin das organische Lösungsmittel aus einem niedrigen Alkanol besteht.

8. Verwendung eines für elektromagnetische Wellen empfindlichen Materials in Form einer für elektromagnetische Wellen empfindlichen Lage, dessen Substratoberfläche mit einer Masse nach einem der Ansprüche 5 bis 7 beschichtet ist.

9. Verwendung eines Materials nach Anspruch 8, worin das polymerisierbare Lipid entsprechend Anspruch 2 definiert ist.

10. Verwendung eines Materials nach Anspruch 8, worin das polymerisierbare Lipid entsprechend Anspruch 3 definiert ist.

11. Verwendung eines Materials nach Anspruch 8, worin R in der allgemeinen Formel II gemäß Anspruch 3 $-(CH_2)_2N^{\oplus}(CH_3)_3$ ist.

12. Verwendung eines für elektromagnetische Wellen empfindlichen Materials nach einem der Ansprüche 1 bis 4 zum Beschichten einer medizinischen Vorrichtung, um sie biokompatibel zu machen.

13. Verwendung eines Materials nach Anspruch 12, wobei ein Polymerisatüberzug gebildet wird, indem man das Material durch Bestrahlen mit einer elektromagnetischen Welle und/oder durch Kontakt mit Sauerstoff einer veinetzenden Polymerisation unterwirft.

## Revendications

1. Utilisation d'un matériau sensible aux ondes électromagnétiques comme composition d'agent de traitement de surface bio-adaptable comprenant un lipide polymérisable possédant comme chaîne acyle hydrophobe au moins une chaine acyle dérivée de l'acide éléostéarique et représentée par la formule I :

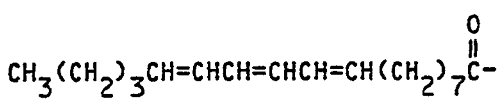

(I)

2. Utilisation d'un matériau selon la revendication 1, selon laquelle ledit lipide polymérisable a comme squelette au moins un élément choisi parmi les suivants : phospholipide, sphingolipide, glycolipide, glycéride, éther de glycérol, phosphate de dialkyle, phosphonate de dialkyle, éther alkylique de phosphinate d'alkyle, phosphonolipide, halogénures d'ammonium N,N-diméthyldisubstitué, halogénures de trialkylméthylammonium, halogénures de tétraalkylammonium, esters dialkyliques de l'acide sulfosuccinique et acide 2,3-diacyloxy succinique.

3. Utilisation d'un matériau selon la revendication 1, selon laquelle ledit lipide polymérisable est un phospholipide de l'acide éléostéarique représenté par la formule générale II :

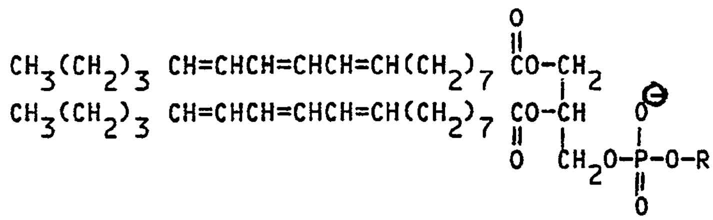 (II)

dans laquelle R est un groupe $-(CH_2)_2-N^{\oplus}(CH_3)_3$, $-(CH_2)_2-N^{\oplus}H_3$ ou $-CH_2CH(N^{\oplus}H_3)-COO^{\ominus}$

4. Utilisation d'un matériau selon la revendication 3, selon laquelle R dans la formule générale II est le groupe $-(CH_2)_2-N^{\oplus}(CH_3)_3$.

5. Utilisation d'un matériau selon l'une quelconque des revendications précédentes sous la forme d'une composition obtenue par dissolution dudit matériau dans un solvant organique.

6. Utilisation d'un matériau selon la revendication 5, selon laquelle la concentration dudit lipide polymérisable dans le solvant organique est comprise dans l'intervalle de 0,1 à 20 %.

7. Utilisation d'un matériau selon la revendication 5, selon laquelle le solvant organique est un alcanol inférieur.

8. Utilisation d'un matériau sensible aux ondes électromagnétiques sous la forme d'une feuille sensible aux ondes électromagnétiques dont la surface du substrat est revêtue d'une composition selon l'une quelconque des revendications 5 à 7.

9. Utilisation d'un matériau selon la revendication 8, selon laquelle le lipide polymérisable est comme défini dans la revendication 2.

10. Utilisation d'un matériau selon la revendication 8, selon laquelle le lipide polymérisable est comme défini à la revendication 3.

11. Utilisation d'un matériau selon la revendication 8, selon laquelle R dans la formule générale II de la revendication 3 est le groupe $-(CH_2)_2-N^{\oplus}(CH_3)_3$.

12. Utilisation d'un matériau sensible aux ondes électromagnétiques selon l'une quelconque des revendications 1 à 4 pour le revêtement d'un appareil médical pour le rendre bioadaptable.

13. Utilisation d'un matériau selon la revendication 12, selon laquelle un revêtement de polymère est formé en soumettant ledit matériau à une polymérisation par réticulation par irradiation à l'aide d'ondes électromagnétiques et/ou par contact avec l'oxygène.

FIG. 1

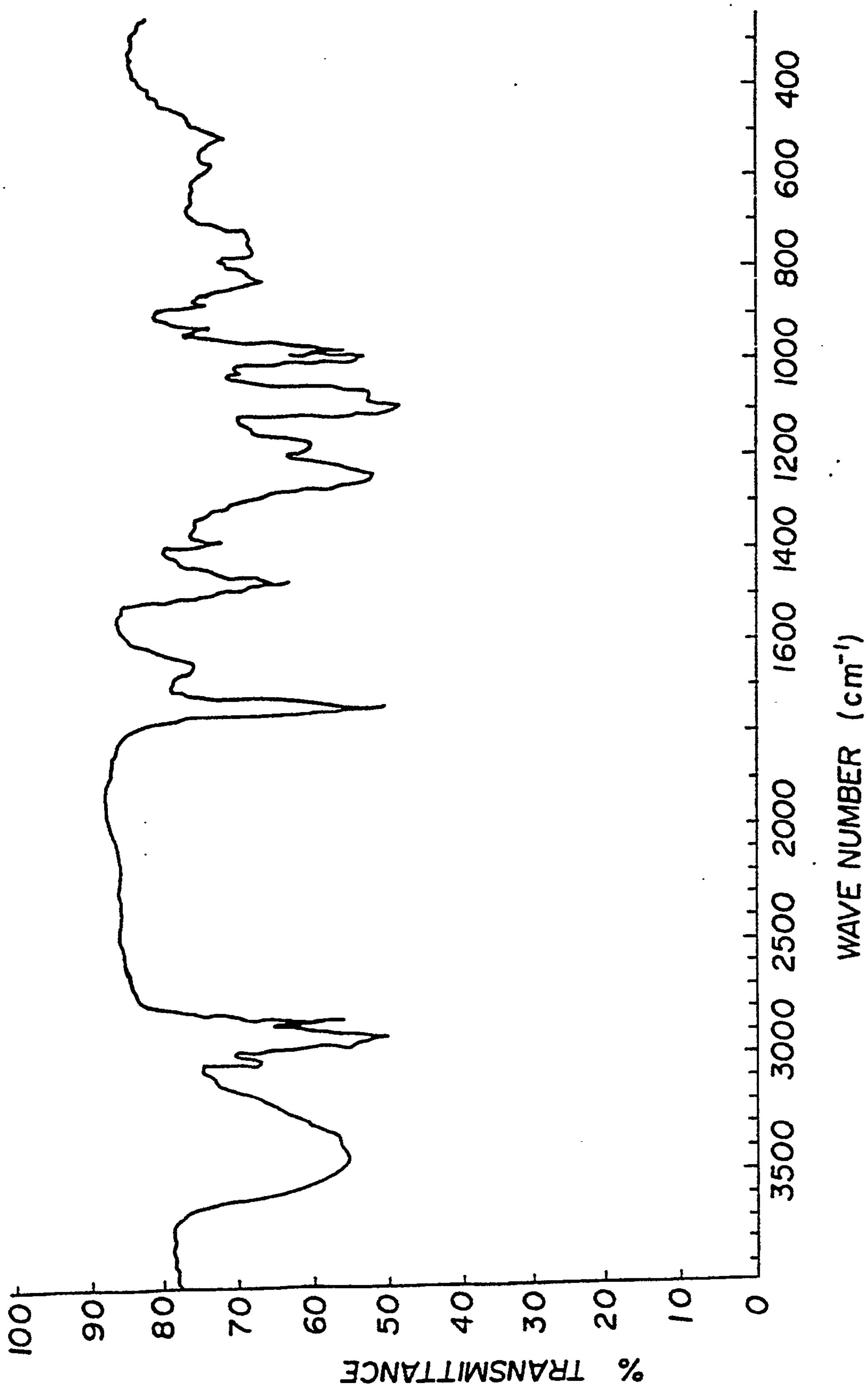

% TRANSMITTANCE
100
90
80
70
60
50
40
30
20
10
0
3500
3000
2500
2000
1600
1400
1200
1000
800
600
400
WAVE NUMBER $(cm^{-1})$

FIG. 2

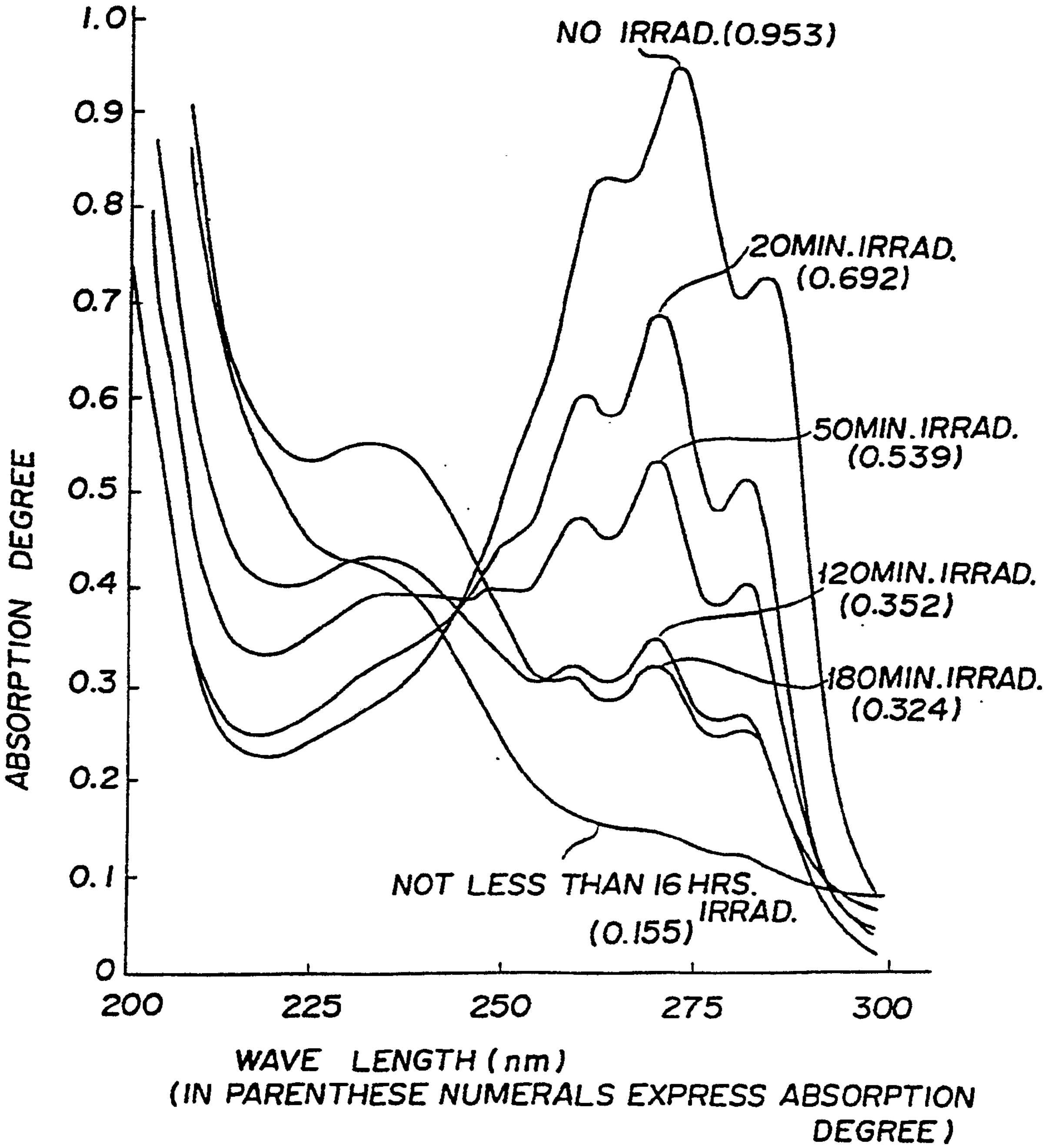
NO IRRAD.(0.953)
20MIN. IRRAD. (0.692)
50MIN. IRRAD. (0.539)
120MIN. IRRAD. (0.352)
180MIN. IRRAD. (0.324)
NOT LESS THAN 16 HRS. IRRAD. (0.155)
ABSORPTION DEGREE
1.0
0.9
0.8
0.7
0.6
0.5
0.4
0.3
0.2
0.1
0
200
225
250
275
300
WAVE LENGTH (nm)
(IN PARENTHESE NUMERALS EXPRESS ABSORPTION DEGREE)